# EUROPEAN PATENT SPECIFICATION

(11) **EP 2 488 506 B1**
(45) Date of publication and mention of the grant of the patent: **29.03.2017**
(21) Application number: 10824252.0
(22) Date of filing: 18.10.2010
(51) Int. Cl.: C07D 279/00, C07D 311/02

(54) **FLUORESCENT CALCIUM INDICATORS THAT ARE RATIOMETRIC AND EMIT IN THE RED SPECTRUM**
RADIOMETRISCHE UND IM ROTSPEKTRUM EMITTIERENDE FLUORESZIERENDE CALCIUM-ANZEIGEN
INDICATEURS DE CALCIUM FLUORESCENTS QUI SONT RATIOMÉTRIQUES ET ÉMETTENT DANS LE SPECTRE ROUGE

(30) Priority: 17.10.2009 US 252654 P
(43) Date of publication of application: 22.08.2012
(73) Proprietor: Asante Research, LLC, Austin, TX 78747 (US)
(72) Inventor: MINTA, Akwasi, Austin, TX 78745 (US); ESCAMILLA, Pedro, Rogelio, Kyle, TX 78640 (US)
(74) Representative: Gee, Steven William
(86) International application number: PCT/US2010/053087
(87) International publication number: WO 2011/047391

(56) References cited:
- US-A- 5 501 980
- US-A1- 2005 233 467
- US-A1- 2007 251 337
- MINTA, A. ET AL.: "Fluorescent Indicators for Cytosolic Calcium Based on Rhodamine andFluorescein Chromophore", THE JOURNAL OF BIOLOGICAL CHEMISTRY, vol. 264, no. 14, 15 May 1989 (1989-05-15) , pages 8171-8178, XP002709965,
- LAVIS LUKE D ET AL: "Bright ideas for chemical biology", ACS CHEMICAL BIOLOGY, AMERICAN CHEMICAL SOCIETY, WASHINGTON, DC, US, vol. 3, no. 3, 1 March 2008 (2008-03-01), pages 142-155, XP002591610, ISSN: 1554-8929, DOI: 10.1021/CB700248M
- YANG ET AL.: 'Seminaphthofluorones are a family of water-soluble, low molecular weight' NIR- EMITTING FLUOROPHORES vol. 105, no. 26, 27 March 2008, DEPARTMENT OF CHEMISTRY, LOUISIANA STATE UNIVERSITY, BATON ROUGE, LA 70803; AND DEPARTMENT OF CHEMISTRY, PORTLAND STATE UNIVERSITY, PORTLAND, OR 97201, pages 8829 - 8834, XP008158014

## Description

### BACKGROUND - FIELD OF INVENTION

This application relates to the design and synthesis of long wavelength fluorescent ion indicators.

### BACKGROUND - PRIOR ART

The most popular fluorescent calcium indicators that are ratiometric include Fura-2 (excitation shifting) and Indo-1 (emission shifting)ⁱ. Ratiometry has the unique advantage of eliminating the variable effects in calcium measurement such as indicator concentration, degree of cell loading, photobleaching, detector sensitivity, cell thickness, and optical path. It is essentially an ideal property that allows for calibration and quantitative measurements. However, these popular ratiometric dyes operate in ultraviolet light. UV optics are expensive and hence have limited the general use of these dyes. The high energy UV light can also be harmful to the cell. The intrinsic fluorescence due to nucleotides and some amino acids when exciting with UV light also causes interference during measurement, although ratiometry does attenuate this problem.

The popular visible wavelength fluorescent ion indicators, Fluo dyesⁱⁱ, do not exhibit ratiometry. They simply markedly enhance emission intensity upon binding calcium. The baseline is not easily delineated, but using manganese or nickel has allowed a useful assessment of the baseline or background in experiments.

There are two ratiometric long wavelength indicators. The first, BTCⁱⁱⁱ, has a very high dissociation constant K_{d} that does not allow for measurement of small changes in cytosolic concentrations (nanomolar) of calcium. The second, Fura-Red^{iv}, has a low quantum yield in water due to its hydrophobicity and therefore has not found much use in cellular studies.

US patent publication 2005/233467 and the paper "Bright Ideas for Chemical Biology" by Lavis, Luke D et al published in ACS Chemical Biology of the American Chemical Society vol. 3, no. 3, 1 March 2008 at pages 142-155, each disclose fluorescent indicators for use in chemical biology.

### SUMMARY OF THE INVENTION

The present invention is a new family of calcium indicators which emit in the red or near infrared of the visible spectrum and shift emission wavelengths on binding calcium, depending on the wavelength of excitation. Exciting at their maximal absorption wavelengths produces an enormous increase in fluorescence, without shift in wavelength, on binding calcium. This new family of calcium indicators is represented by four distinct compounds and their variants.

The first of these compounds comprises a BAPTA backbone conjugated via carbon-carbon bond to a fluorophore, either a putative seminaphthofuorescein or seminaphthorhodamine. The emission maxima of these dyes are in the red at 650 nm.

The pKₐ of the seminaphthofuorescein and seminaphthorhodamine is reduced by halogenation of the naphthol moiety with fluorines and chlorines. Thus, the pKₐ of the nonhalogenated seminaphthofluorescein is reduced from 7.8 to 5.5 via di-orthohalogenation, making the dies unresponsive to small pH changes in the cell.

### Compound I:

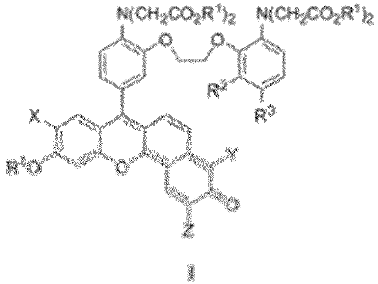

X, Y, and Z are any combination of H, F, or Cl
R¹ = H, a salt (i.e., K⁺), or CH₂OCOCH₃ (AM ester)

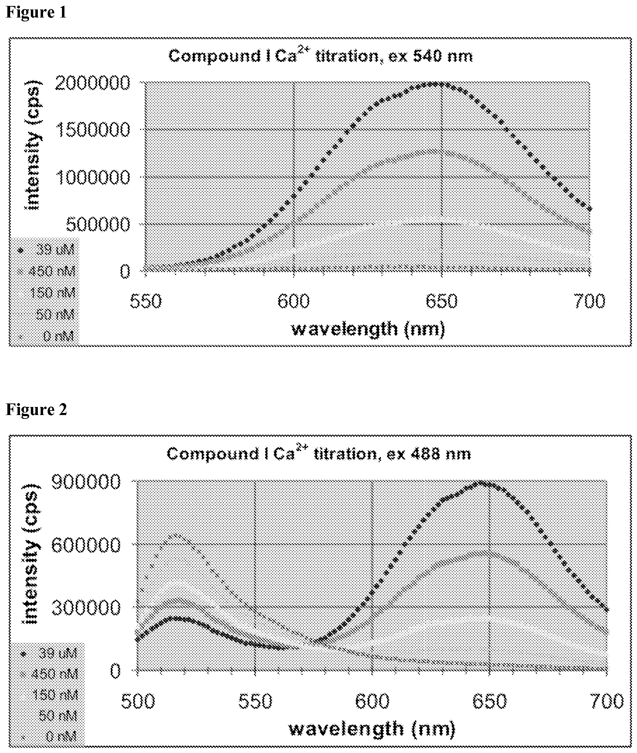

Compound I has dissociation constants between 300 nM and 400 nM and pKₐ's of about 5.5. Compound I has and excitation maximum of 540 nm and an emission maximum of 650 nm (Figure 1). However, when excited at 488 nm, it shows a calcium-dependent emission shift from 525 nm to 650 nm that makes it ratioable (Figure 2).

Compound III has a unique chelating backbone. The BAPTA has an extra carboxylic function ortho to the fluorophore. The dye conjugated to this BAPTA most closely resembles 6-aminoseminaphthorhodamine for an enhanced quantum yield. The naphthols have chlorine or fluorine substitution to reduce the pKa from greater than 8 to 5.5. The emission maximum is in the red at 650 nm.

### Compound III:

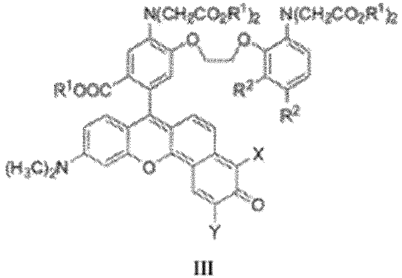

X and Y are any combination of H, F, or Cl
R¹ = H, a salt (i.e., K⁺), or CH₂OCOCH₃ (AM ester)

Compound IV has the same unique chelating backbone as Compound III, with an carboxylic function ortho to the fluorophore, closely resembling 6-aminoseminaphthofluorescein. The naphthols have chlorine or fluorine substitution to reduce the pKₐ from greater than 8 to 5.5. The emission is maximum is 650 nm.

### Compound IV:

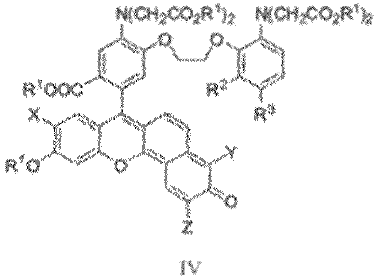

X and Y are any combination of H, F, or Cl
R¹ = H, a salt (i.e., K⁺), or CH₂OCOCH₃ (AM ester)

These compounds have been modified at the R² and R³ positions to provide variations which give them special functions in the cell as needed.
a. When R² is H and R³ is methyl, the default high affinity version results.
b. When R³ is modified as CH₂CH₂CO₂R¹, a new leakage resistant version results, without changing the fiuorescent properties.
c. When R² and R³ are F, a low affinity version results, with a K_{d} for calcium almost twenty times higher than the default high affinity version, also without changing the fiuorescent properties.
d. When 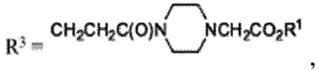 a mitochondrial version results, allowing calcium measurement in mitochondria rather than in the cytosol.
e. When 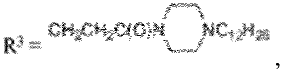 a near-membrane version results, allowing calcium measurement near the membrane without affecting fluorescent properties.

These variations extend the utility of the new inventions for calcium studies in the cell.

### BRIEF DESCRIPTION OF DRAWINGS

These and other objects and advantages of the present invention are set forth below and further made clear by reference to the drawings therein:
Figure 1 is a typical titration of compound I with excitation at 540 nm emission at 650 nm.
Figure 2 is a typical titration of compound I with excitation at 488 nm showing emission at 525 nm and 650 nm.
Figure 3 is a typical titration of an alternative compound which is not according to the present invention, with excitation at 635 nm and emission at 690 nm.
Figure 4 is the response of compound I to calcium in neuron cells.
Figure 5 is a synthesis scheme of the naphthalene diol precursor with diortho chloro/ fluoro halogenation.
Figure 6 is a Method A synthesis scheme for compound I.
Figure 7 is a Method B synthesis scheme for an alternative compound which is not according to the present invention.
Figure 8 is a Method C synthesis scheme for compounds III and IV.
Figure 9 is a synthesis scheme of compound I.

### DETAILED DESCRIPTION OF DRAWINGS

### Definition of terms used herein

In the present specifications and claims, reference will be made to phrases and terms of art which are expressly defined for use herein as follows:
As used herein [Ca²⁺] means intracellular free calcium.
As used herein EGTA means ethylene glycol bis- (-beta-aminoethylether_-N,N, N', N' tetraacetic acid.
As used herein "BAPTA-like" means substituted derivatives of BAPTA which retain the essential characteristics of the two bis(carboxyethyl) amino-substituted phenyl rings, said rings being linked at the positions ortho to the amines through a four atom bridge wherein the atom adjacent to each phenyl ring is O and the two center atoms are each carbon. The 6-position on one of the phenyl rings linked to the fluorophore may be a carboxylic function or H.
As used herein AM ester means acetoxymethyl ester, or any ester form to facilitate cell loading.
As used herein "µM" means 10⁻⁶ moles/ liter and "nM" means 10⁻⁹ moles/ liter.
As used herein MOPS means 3-(N-morpholino)propanesulfonic acid.

### Brief description of invention

The present invention introduces a new family of calcium indicators with emissions in the red and near infrared regions of the electromagnetic spectrum. The new indicators are derived from a BAPTA or BAPTA-like backbone for calcium chelation, and the red or infrared fluorescence is achieved by linking the chelating portion with a chlorinated and fluorinated Benzo[c]xanthenone. When the xanthenone is derived from a combination of resorcinol and naphthalene diol, the omission is 650 nm, and a derivation from two napthalene diols gives an emission of 690 nm. As calcium indicators they respond to intracellular calcium. The longer wavelength causes a slight reduction of quantum yield.

### Detailed description

Figure 1 shows a non-ratiometric calcium titration of Compound I. Appropriate volumes of 10 mM potassium-EGTA, 100 mM KCl, 10 mM MOPS buffer at pH 7.20 and 10 mM calcium-EGTA, 100 mM KCl, 10 mM MOPS buffer at pH 7.20, each containing 5 µM Compound I were mixed to give the desired free calcium concentrations of 0 nM, 50 nM, 150 nM, 450 nM, and 39 µM. Emission spectra were recorded of each solution with excitation at 540 nm, resulting in a strong increase in emission intensity at 650 nm with increasing calcium concentration. Calculation of dissociation constant gave approximately 400 nM. Spectra were recorded on SPEX Fluoromax-3.

Figure 2 shows a ratiometric calcium titration of Compound I. Appropriate volumes of 10 mM potassium-EGTA, 100 mM KCl, 10 mM MOPS buffer at pH 7.20 and 10 mM calcium-EGTA, 100 mM KCl, 10 mM MOPS buffer at pH 7.20, each containing 5 µM Compound I were mixed to give the desired free calcium concentrations of 0 nM, 50 nM, 150 nM, 450 nM, and 39 µM. Emission spectra were recorded of each solution with excitation at 488 nm, resulting in decreasing emission intensity at 520 nm and increasing emission intensity at 650 nm, both with increasing calcium concentration. The isosbestic point occurs at 575 nm. Spectra were recorded on SPEX Fluormax-3.

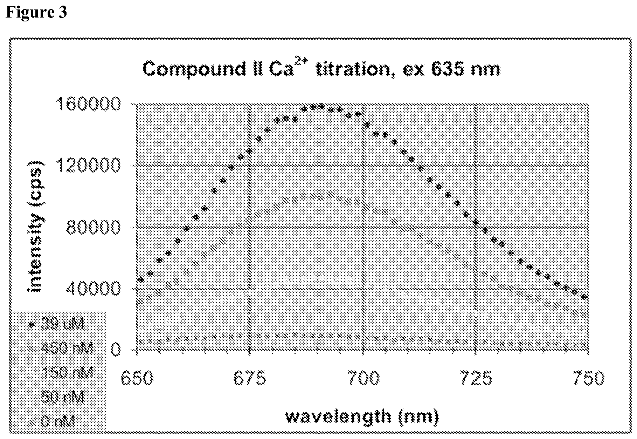

Figure 3 shows a nonratiometric calcium titration of an alternative compound. Appropriate volumes of 10 mM potassium-EGTA, 100 mM KCl, 10 mM MOPS buffer at pH 7.20 and 10 mM calcium- EGTA, 100 mM KCl, 10 mM MOPS buffer at pH 7.20, each containing 5 µM Compound I were mixed to give the desired free calcium concentrations of 0 nM, 50 nM, 150 nM, 450 nM, and 39 µM. Emission spectra were recorded of each solution with excitation at 635 nm, resulting in increasing emission intensity at 690 nm with increasing calcium concentration. Spectra were recorded on a SPEX Fluormax-3.

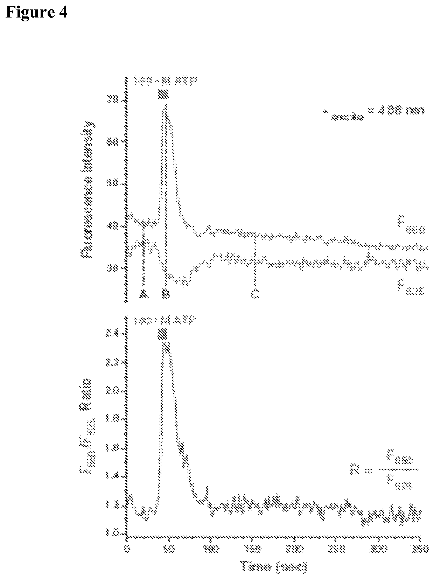

Figure 4 shows the response of Compound I to calcium in rat vagal neurons. It is an emission ratiometric measurement of ATP-evoked Ca²⁺ transients in rat vagal neurons. The inferior vaga (nodose) ganglia from a Sprague-Dawley rat were dissociated enzymatically. The yield of nodose neurons was suspended in Liebowitz L-15 medium supplemented with 10% ( v/v) fetal bovine serum and penicillin- streptomycin and plated onto No. 1 glass cover slips. The neurons were incubated for 50 minutes at room temperature with 3 µM Compound I, AM ester. Thereafter, the cells were washed and maintained in fresh L-15 medium for 40 minutes to permit intracellular hydrolysis of the AM ester to proceed to completion. The indicator-loaded neurons were positioned on the stage of an inverted microscope (Axiovert 100M Zeiss) and superfused with Locke solution (equilibrated with a mixture of 5% CO2 and 95% 02). Confocal imaging microscopy was performed with LSM 510 system (Zeiss) through a 9 x 40 objective (N.A. 1.2; oil- immersion) at a frame rate of 0.5 Hz. Excitation was at 488 nm; a 545 nm dichroic mirror separated the florescence emission into two components: a short wavelength component (designated F525) that passed through a 500-550 nm band-pass filter, and a long wavelength component (F650) that passed through 560 nm long pass filter. Neurons were stimulated with a 10-sec pulse of 100 um ATP delivered through the superfusate.

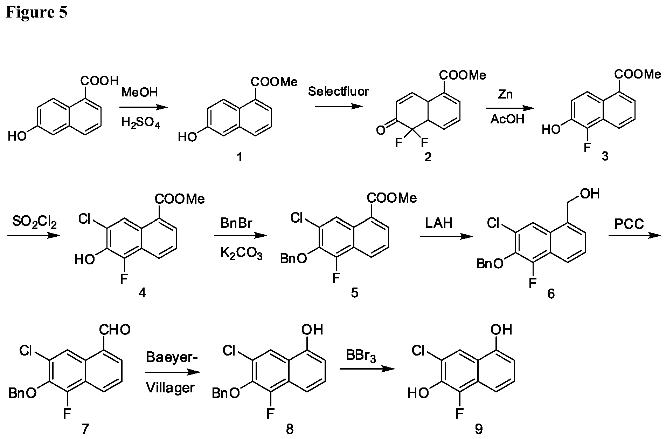

Figure 5 shows the synthetic scheme for the synthesis of the halogenated napthol precursor 6-hydroxynapthoic acid was esterified and the resulting ester was geminally difluorinated with Selectfluor to give (2), immediately followed by reduction with zinc and acetic acid to give the monofluoro compound (3); the fluorinated compound was chlorinated to give (4) and the phenol was then protected with benzyl bromide to give (5). The final compound was obtained by first reducing the methyl ester of (5) with lithium aluminum hydride to alcohol (6) and oxidizing the alcohol to aldehyde (7) with pyridinium chlorochromate. The aldehyde was converted to napthol (8) by Baeyer-Villiger reaction and (9) was obtained by deprotection of the benzyl protection with BBᵣ₃.

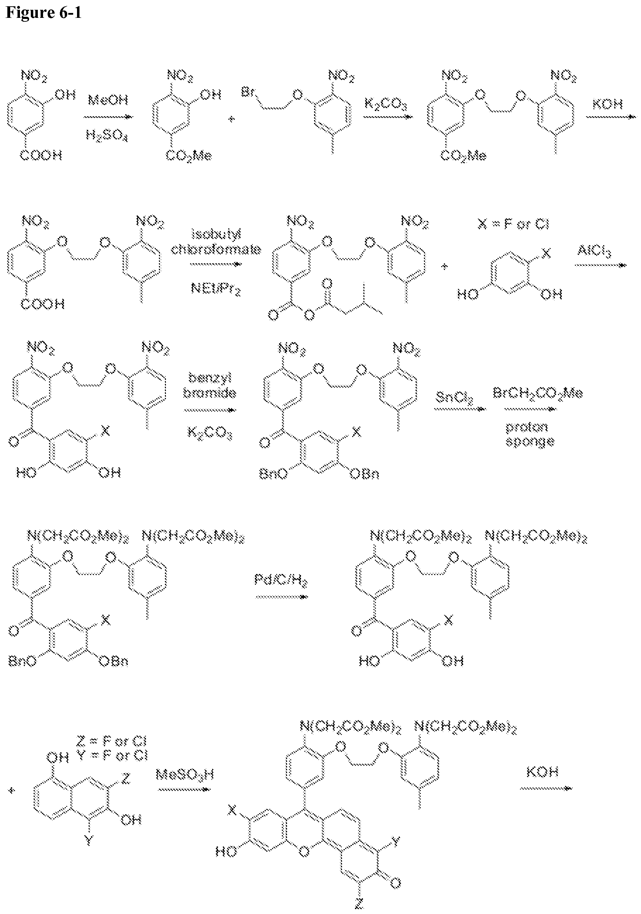

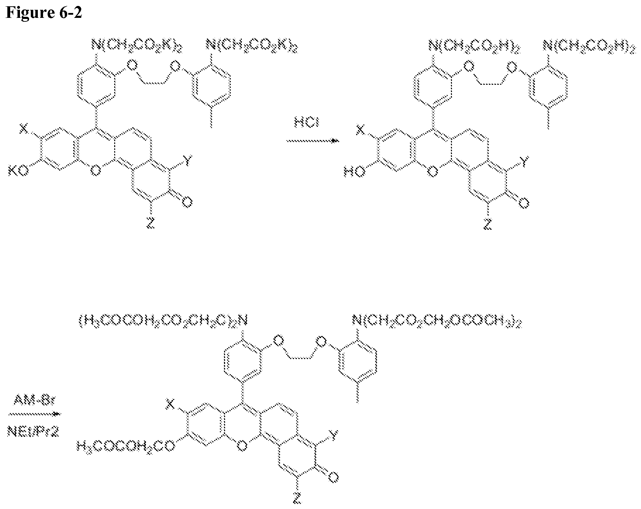

Figure 6 shows a Method A for the synthesis of Compound I.
Commercially available 3-hydroxy-4-nitrobenzoic acid was esterified and then coupled with 2-bromoethoxy-4-methyl-nitrobenzene to form the BAPTA backbone. After hydrolyzing the ester to the free acid, the mixed anhydride was formed and reacted with 4-haloresorcinol to give the benzophenone. The phenol groups were protected as benzyl ethers prior to reducing the nitro groups with stannous chloride and alkylating the resulting anilines. Debenzylating with palladium and hydrogen prepared the ketone to form dye by coupling with 5,7-dihalo-1,6-dihydroxynaphthalene in the presence of methanesulfonic acid. The dye was subsequently hydrolyzed to the calcium-chelating potassium salt. Acidification gave the free acid, which was then alkylated with bromomethyl acetate to give the membrane permeable acetoxymethyl (AM) ester.

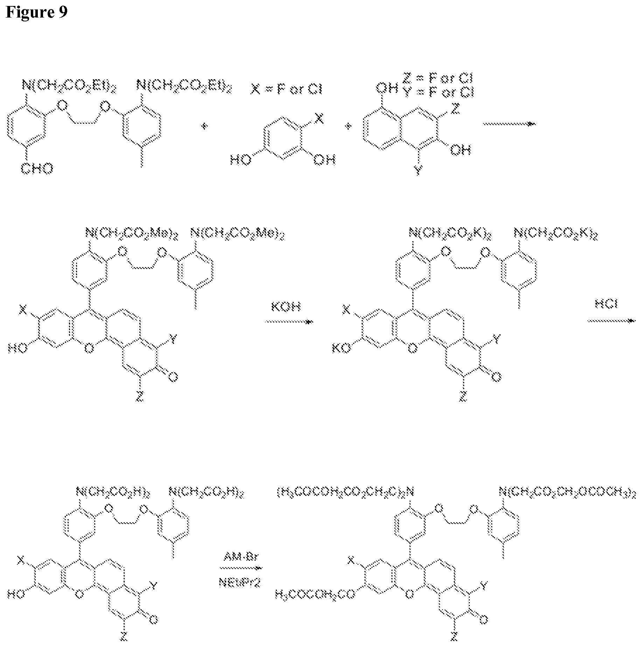

Figure 9 shows Method B for synthesis of Compound I.
The aldehyde precursor from Grynkiewicz et al was coupled to 4-haloresorcinol and 5,7-dihalo-1,6-dihydroxynaphthalene in the presence of methanesulfonic acid. The ester was then hydrolyzed to the calcium-chelating potassium salt. Acidification gave the free acid which was alkylated to give the acetoxymethyl (AM) form.

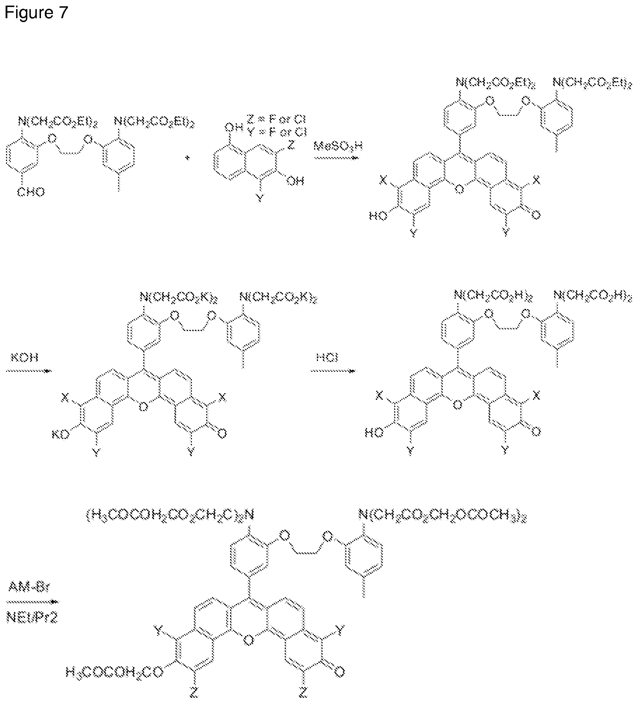

Figure 7 shows a Method B for synthesis of an alternative compound.
The aldehyde precursor from Grynkewicz et al¹ was coupled to 5,7-dihalo-1,6-dihydroxynaphthalene in the presence of methanesulfonic acid. The ester was then hydrolyzed to the calcium-chelating potassium salt. Acidification gave the free acid which was alkylated to give the acetoxymethyl (AM) form.

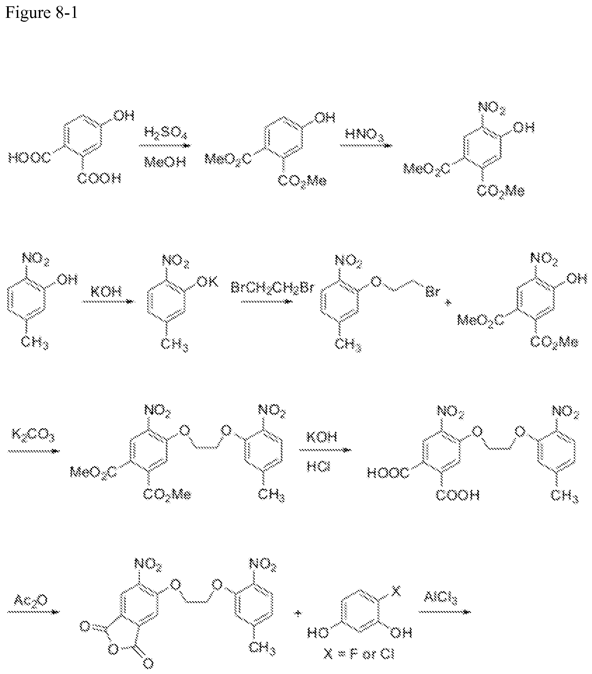

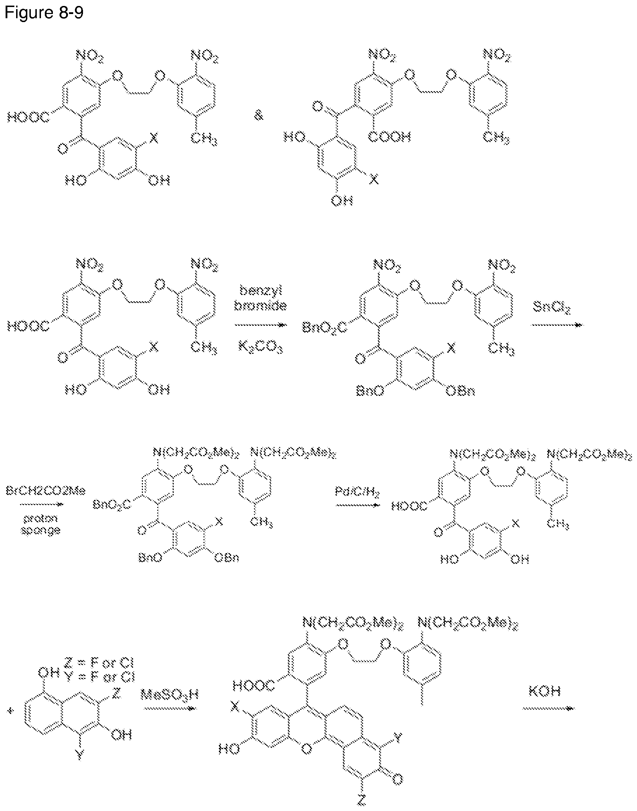

Figure 8 shows a method C for the synthesis of compounds III and IV.
Commercially available 4-hydroxyphthalic acid was esterified to the dimethyl phthalate prior to nitration at the 5 position. Reaction with 2-bromoethoxy-4-methyl-nitrobenzene yielded the BAPTA backbone. After hydrolyzing the methyl esters, the phthalic acid in acetic anhydride formed the cyclic anhydride. Friedel-crafts acylation with 4-halo resorcinol gave the isomeric benzophenones, which were separated by column chromatography before proceeding further. The phenols and free acid were benzylated prior to reducing the nitro groups with stannous chloride, followed by alkylation of the resulting anilines. Debenzylation with palladium and hydrogen allowed dye formation when coupled to 5,7-dihalo-1,6-dihydroxynaphthalene in the presence of methanesulfonic acid. The dye was hydrolyzed to the calcium-chelating potassium salt. Acidification yielded the free acid, which was alkylated to give the membrane-permeable AM form.

### Synthesis of compound

One aspect of the invention is the ability to lower the pKₐ of the seminaphthofluorescein, dinaphtho fluorescein, and seminaphthorhodamine-like fluorophores. The precursor that enabled such a change is 5-fluoro-7-chloro-1,6-naphthalene diol (Figure 5, compound 9).

### Synthesis of Compound 9 from Figure 5 Experimental:

### Methyl-5-fluoro-6-hydoxynaphthoate (3)

6-hydoxynaphthoic acid (25 g) was dissolved in methanol (250 ml) and adding sulfuric acid (25ml). The mixture was heated at 80°C overnight. The reaction mixture was allowed to cool and diluted with ethyl acetate and washed with brine, then sodium bicarbonate, and dried over sodium sulfate. Evaporation of the solvent gave the ester as a creamy solid (24g). The ester (1Og) was dissolved in acetonitrile (500 ml) and two equivalents of Selectfluor were added and stirred at room temperature for 3 hours at which time the starting material was consumed. The reaction mixture was diluted with ethyl acetate, washed with brine, and dried over sodium sulfate to give geminally difluorinated compound (2) as cream-colored solid (7g). This solid was reduced to the monofluoro compound with 2 g zinc powder in 200 mL acetic acid. The solids were filtered and the solvents removed under reduced pressure. Column chromatography (6: 1 hexanes/ethyl acetate) gave 6 g of compound 3.

### Methyl- 5-fluoro-6-benzyloxy-7-chloro-naphthoic ester (5)

Compound (3) 5g was dissolved and toluene (50ml) and sec-butylamine (500 µ 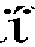 ) was added and the reaction mixture kept at 66°C on an oil bath and sulfuryl chloride (1.2 equivalents) was added over 3 hours. The mixture was diluted with ethyl acetate and washed with brine, the 5% sodium thiosulfate dried over sodium sulfate and evaporated to give (4). The residue was dissolved in acetonitrile (10 ml), and potassium carbonate (4g), followed by benzyl bromide (3 ml), were added. The mixture was heated under reflux for two hours when TLC showed all starting material was consumed. The reaction mixture was diluted with ethyl acetate and washed with brine, dried and evaporated to get oily residue which was purified by column chromatography in 9: 1 hexane to ethyl acetate to give (5) as white solid (3g).

### 5-fluoro-6-benzydoxy- 7-chdoro-naphthadedhyde (6)

Compound (5) (5.8 g) was dissolved in diethylether (100 ml) and added dropwise to lithium aluminum hydride (2 equivalents) in 75 mL of dry ether. When addition was complete, stirring was continued for another 30 minutes, and the reaction mixture was diluted with ethyl acetate and stirred with dilute sulfuric acid. The organic extract was dried and evaporated to give a creamy residue. The residue was dissolved and dry methylene chloride (75 ml) and added to pyridinium chlorochromate (1.5 equivalent) in CH2CI2 (75 ml) and stirred for 2 hours at room temperature. Ethyl acetate was added to the reaction mixture, which was then filtered through a 300 cm³ of silica. Evaporation of the organic filtrate gave (6) as 4g of a light brown solid.

### 5-fluoro-6benzydoxy- 7-chdoronapthol (8)

Compound 6 (3g) was dissolved in benzene (30 ml), and m-chloroperbenzoic acid (2 equivalents) was added, and the mixture was heated at 70° C for 24 hours. 10 mis of 1M KOH were added as the mixture stirred to hydrolyze the formate ester. The reaction mixture was acidified with 1M HC1 and extracted with ethyl acetate, washed with brine, dried over sodium sulfate and evaporated to give a gummy residue, purified by column chromatography in 4:1 hexane to ethyl acetate to give (8) as creamy solid 2g.

### 5-fluoro-7-chdoro-l,6-naphthalene diol (9)

Compound 8 (2g) was dissolved in methylene chloride (20 mis), boron tribromide (1M in CH2C12, three equivalents) was added, and the solution was stirred for three hours. Addition of ice water to the reaction and extraction with methylene chloride, followed by ethyl acetate gave (9) after washing and evaporation as light brown solid (1 g) . It was ready for use directly for synthesis of the dyes.

### Synthesis of Compound I Experimental:

### Compound I, Ethyl ester:

BAPTA aldehyde from Grynkewicz et al¹ (1 mmol), 4-fluororesorcinol (1 mmol), and 5-fluoro-7-chloro-1,6-naphthalene diol (1 mmol) were dissolved in 10 mL methanesulfonic acid at room temperature for one hour. The reaction was poured into 200 mL 3M sodium acetate in ice, and the violet precipitate was filtered. After drying under vacuum over phosphorous pentoxide, the solid was dissolved in 20 mL 1 : 1 chloroform/methanol and p-chloranil (2 equivalents) was added. The solution was refluxed overnight. The reaction mixture was evaporated to dryness, and then the violet solid was redissolved in methanol. Insoluble p-chloranil was filtered off. The filtrate was evaporated and purified by column chromatography using a 5-15% methanol/chloroform gradient to give Compound I, Ethyl ester.

### Compound I, Pentapotassium Salt:

Pure Compound I, Methyl ester (0.5 mmol) was hydrolyzed with 2 M aqueous KOH (4.0 mmol) in 4 mL methanol at room temperature for thirty minutes. 2 mL water were added, and the methanol was removed under reduced pressure. The fully aqueous reaction was stirred at room temperature for one hour, at which point C₁₈ TLC (3:2 methanol/brine) showed the hydrolysis was complete. Ice was added to the reaction mixture, and 2M HC1 was added until the pH was 7.5 to 8.5. The mildly basic solution was loaded onto 30 g of LH-20 column packed in water and eluted with water. Pure product fractions were stripped of solvent at 45 °C under vacuum to give Compound I, Pentapotassium Salt.

### Compound I, Acetoxymethyl (AM) Ester:

Compound I, Pentapotassium Salt (0.1 mmol) was dissolved in 1 mL ice water. 2M HC1 was added to bring the pH to 1.5, and the precipitated free acid was filtered. The violet solid was dried under vacuum over phosphorous pentoxide. It was then dissolved in 1 mL dry dimethylformamide, and 1.5 mmol diisopropylethylamine were added. After stirring for five minutes at room temperature, 1 mmol bromomethyl acetate was added, and the reaction was stirred at room temperature for two hours. The mixture was diluted with 50 mL ethyl acetate and washed twice with 50 mL 0.1M citric acid and once with 50 mL brine. The organics were dried over sodium sulfate, filtered, and stripped free of solvents under reduced pressure. Column chromatography (1 : 1 hexanes/ethyl acetate) gave pure Compound I, AM ester.

### References

### Patents

U.S. Patent No. 4,849,362; DeMarinis et al July 1989.
U.S. Patent No. 5,049,673; Tsien et al September 1989.
U.S. Patent No. 4,603,209; Tsien et al July 1986.

### Publications

i Grynkiewicz, G., Poenie, M., Tsien, R.Y., J. Biol. Chem. (1985) 260, 3440-3450.
ii Minta, A., Kao, J., Tsien, R.Y., J. Biol. Chem. (1989) 264, 8171-8178.
iii latridou, H., Foukaraki, I., Kuhn, M. A., Marcus, E. M., Haugland, R. P., Katerinopoulos, H.E. Cell Calcium, (1994) 15, 190-198.
iv DeMarinis, R. M., Katerinopoulos, H.E., Muirhead, K. A. Biochem. Methods (1990) 112, 381

## Claims

1. A chemical compound having the general formula: 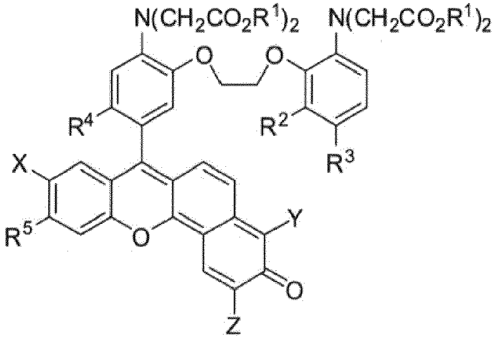 and the pharmaceutically acceptable non-toxic salts and esters thereof wherein:
R¹ is H, a salt, or CH₂OCOCH₃;
R² is H, F, or Cl
R³ is H, Me, F, CH₂CH₂CO₂R¹, 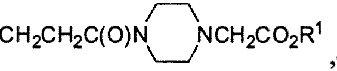 or 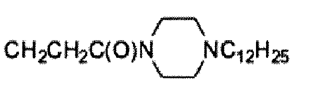
R⁴ is H or CO₂R₁
R⁵ is OR₁ or N(CH₃)₂
X, Y, and Z are independently H, F, or Cl.

## Patentansprüche

1. Chemische Verbindung mit der allgemeinen Formel: 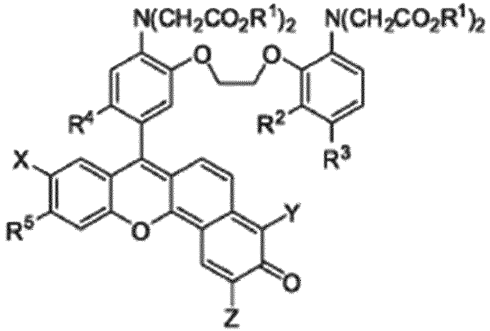 und die pharmazeutisch akzeptablen nicht-toxischen Salze und Ester davon, wobei:
R¹ H, ein Salz oder CH₂OCOCH₃ ist;
R² H. F oder Cl ist
R³ H, Me, F, CH₂CH₂CO₂R¹, 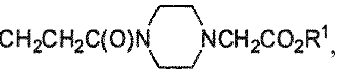 oder 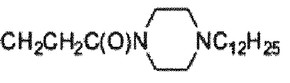 ist
R⁴ H oder CO₂R₁ ist
R⁵ OR₁ oder N(CH₃)₂ ist
X, Y und Z unabhängig voneinander H, F oder Cl sind.

## Revendications

1. Un composé chimique ayant la formule générale : 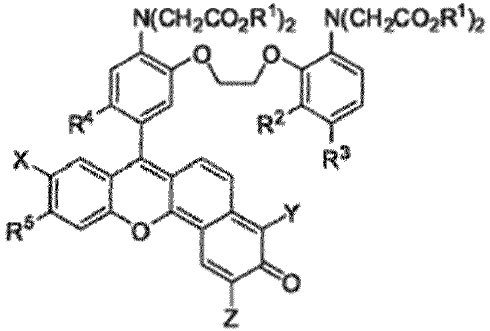 et les sels non toxiques pharmaceutiquement acceptables et les esters de ceux-ci où :
R¹ est H, un sel, ou CH₂OCOCH₃ ;
R² est H, F, ou Cl
R³ est H, Me, F, CH₂CH₂CO₂R¹, 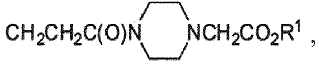 ou 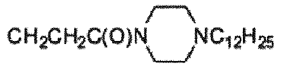
R⁴ est H ou CO₂R₁
R⁵ est OR₁ ou N(CH₃)₂
X, Y, et Z sont indépendamment H, F, ou Cl.
